# EUROPEAN PATENT SPECIFICATION

(11) **EP 1 761 262 B1**
(45) Date of publication and mention of the grant of the patent: **03.12.2008**
(21) Application number: 05756055.9
(22) Date of filing: 17.06.2005
(51) Int. Cl.: A61K 31/42, C07D 261/20

(54) **MARINE COMPOUNDS WITH CALCIUM CHANNEL BLOCKING PROPERTIES FOR THE TREATMENT OF COGNITIVE OR NEURODEGENERATIVE DISEASES**
VERBINDUNGEN MARITIMEN URSPRUNGS MIT KALZIUM-KANAL-BLOCKIERENDEN EIGENSCHAFTEN ZUR BEHANDLUNG KOGNITIVER ODER NEURODEGENERATIVER ERKRANKUNGEN
COMPOSES MARINS POSSEDANT DES PROPRIETES BLOQUANT LES CANAUX CALCIQUES, DESTINES AU TRAITEMENT DE MALADIES COGNITIVES OU NEURODEGENERATIVES

(30) Priority: 17.06.2004 EP 04076731
(43) Date of publication of application: 14.03.2007
(73) Proprietor: Neuropharma, S.A., 28760 Madrid (ES)
(72) Inventor: MARTÍNEZ GIL, Ana, E-28004 Madrid (ES); ALONSO GORDILLO, Diana, E-28230 Las Rozas - Madrid (ES); DORRONSORO DÍAZ, Isabel, E-28005 Madrid (ES); GARCÍA PALOMERO, Esther, 28911 Leganés - Madrid (ES); DE AUSTRIA DE LUQUE, Celia, E-28030 Madrid (ES); USÁN EGEA, Paola, E-28430 Alpedrete - Madrid (ES); DEL MONTE MILLÁN, María, E-28032 Madrid (ES); MEDINA PADILLA, Miguel, E-28035 Madrid (ES)
(74) Representative: ABG Patentes, S.L.
(86) International application number: PCT/EP2005/006643
(87) International publication number: WO 2005/123073

(56) References cited:
- WO-A-92/17475
- CIMINIELLO P ET AL: "Chemistry of Verongida Sponges VIII - Bromocompounds from the Mediterranean Sponges Aplysina aerophoba and Aplysina cavernicola" TETRAHEDRON, ELSEVIER SCIENCE PUBLISHERS, AMSTERDAM, NL, vol. 53, no. 18, 5 May 1997 (1997-05-05), pages 6565-6572, XP004105650 ISSN: 0040-4020
- MORRIS S A ET AL: "NITROGENOUS METABOLITES FROM THE DEEP WATER SPONGE HEXADELLA SP" CANADIAN JOURNAL OF CHEMISTRY, NATIONAL RESEARCH COUNCIL. OTTAWA, CA, vol. 67, no. 4, 1989, pages 677-681, XP009001184 ISSN: 0008-4042 cited in the application

## Description

### FIELD OF THE INVENTION

This invention relates to compounds obtained from marine sponges, particularly *Aplysina sp.* and *Oceanapia,* and more particularly to spirocompounds isolated from the mediterranean sponges *Aplysina cavernicola, Aplysina fulva* and *Oceanapia,* and their use in the treatment of cognitive or neurodegenerative diseases or disorders.

### BACKGROUND OF THE INVENTION

VDCC blockers (block voltage-dependent calcium channel) are traditionally used for treatment of cardiovascular diseases, but these drugs may also be applied for pain treatment or for some neurodegenerative disorders, for instance Alzheimer's disease (AD) (Verkhratsky et al., 2003, J Cell Mol Med, 7, 351-61).

AD is an irreversible disorder, characterized by a progressive loss of several cognitive abilities. Although many factors have been implicated in this type of dementia, its etiology and pathogenesis remains unclear. One of the characteristic changes in the AD brain is the deficit of cerebral acetylcholine. The "cholinergic hypothesis" (Winkler et al., 1998, J Mol Med, 76, 555-67) represents one of the most useful approaches involved in treatment of AD, and led to development of drugs with an AChE (acetylcholinesterase) inhibition activity. AChE expression is upregulated around amyloid plaques in AD patients, but this mechanism is unknown, although there are evidences suggesting that β-amyloid could increase AChE expression by increasing intracellular calcium involving the opening of VDCC (Sbema et al., 1997, J Neurochem, 69, 1177-84). There is also emerging evidence showing that an altered calcium homeostasis can play an important role in this pathology (Mattson et al., 2000, Trends Neurosci, 23, 222-9).

The oceans are the source of a large group of structurally unique natural products that are mainly accumulated in invertebrates such as sponges, tunicates, bryozoans, and mollusc (Burkhard et al., 2003, Drug Dis Today, 8, 2, 536-544). Several of these compounds show pronounced pharmacological activities and are interesting candidates for new drugs in several areas of treatment (Newman et al., 2003, J Nat Prod, 66, 1022-1037). Once again sponges have provided more marine natural products than any order phylum, due in part to their propensity to produce bioactive metabolites (Faulkner et al., 2002, Nat Prod Rep, 19, 1-48).

There is still a need for new compounds able to modulate VDCC, which could be useful in the treatment of cognitive or neurodegenerative diseases. With this aim, a screening of marine compounds was performed on SH-SY5Y human neuroblastoma cells exposed to KCI 120 mM, in which survival is tested 24 hours later.

### SUMMARY OF THE INVENTION

In a first aspect, the present invention provides the use of a family of compounds of formula I as defined in the claims, some of them present in the marine sponges *Aplysina cavernicola, Aplysina fulva* and *Oceanapia,* which block VDCC, in the preparation of a medicament for the treatment of cognitive, neurodegenerative and prion diseases or disorders. Some of these compounds additionally inhibit AChE and BuChE.

These compounds are, consequently, useful for the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimer's disease or condition, or prion disease as Creutzfeld-Jakob disease or Gerstmann-Straussler-Scheinker disease.

A further aspect of the invention relates to a compound of formula:

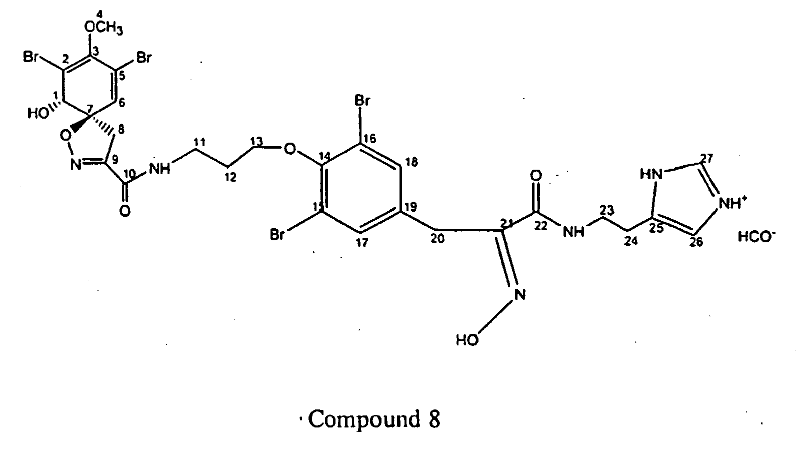

In another aspect, the invention is related to a pharmaceutical composition which comprises Compound 8 as defined above, or a tautomer, enantiomer, diastereoisomer or a different pharmaceutically acceptable salt, a prodrug or a solvate therof, together with a pharmaceutically acceptable carrier, adjuvant or vehicle.

Another aspect of the invention is related to processes either for preparing or for isolating from the marine sponges *Aplysina cavernicola* or a mixture of *Aplysina fulva* and *Oceanapia* the compounds of formulas 3 to 8.

Finally, the present invention is also directed to organic solvent extracts of *Aplysina cavernicola*, to said extracts for use as medicaments, in particular for the treatment of cognitive disorders, to the use of said extracts in the manufacture of a medicament, in particular for the treatment of cognitive disorders, and to a process for obtaining said extracts.

### DETAILED DESCRIPTION OF THE INVENTION

Organic solvent extracts, in particular the isopropanolic extract, of the marine sponges *Aplysina cavernicola* and a mixture of *Aplysina fulva* and *Oceanapia* showed VDCC blocker activity and some additionally showed AChE and BuChE enzymes inhibition.

Those compounds that exhibited a neuroprotective effect were selected and further assayed in SH-SY5Y cells loaded with Fluo-4 to measure their effects on changes in intracellular Ca²⁺ in response to depolarization with KCl. The AChE and BuChE inhibitory activities of some of the compounds have also been evaluated following the Ellman's method (Ellman's et al., 1961, Biochem Pharmacol, 7, 88-95). All of them showed inhibition of these enzymatic activities in the 10⁻⁵ M range.

After careful investigations, the authors of the invention have found that a family of compounds, some of them present in the marine sponges *Ap*/*ysina cavernicola, Aplysina Fulva* and *Oceanapia,* block VDCC; some of the compounds additionally inhibit AChE and BuChE. The property of blocking VDCC could be a good approach in the treatment of AD and some other cognitive and neurodegenerative diseases. The combination of this property with the inhibition of AchE and BuChE could be even a better approach.

The present invention provides, as stated above, the use of a structurally distinct family of compounds of formula I, some of them present in the marine sponges *Aplysina cavernicola, Aplysina Fulva* and *Oceanapia,* which block VDCC and some additionally show AChE and BuChE inhibition, in the manufacture of a medicament for the treatment of cognitive, neurodegenerative and prion diseases.

Cognitive, neurodegenerative and prion diseases within the meaning of the present invention are, but not limited to, cognitive disorders as senile dementia, cerebrovascular dementia, mild recognition impairment, attention deficit disorder, and/or neurodegenerative dementing disease with aberrant protein aggregations as specially Alzheimer's disease or condition, stroke or prion disease as Creutzfeld-Jakob disease or Gerstmann-Straussler-Scheinker disease.

Characteristic of the compounds is the presence of a heterocyclic unit comprising a spiro ring connected to another spiro ring, an imidazole ring or an amide group through a linker of a length between a certain range. The compounds are represented by formula I: 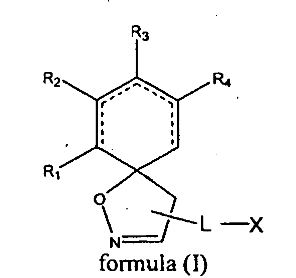 wherein
L is a linker, consisting of a lineal sequence of 3-20 units selected from the group formed by -CR₆R₇-, -CR₆=, =CR₆-, -CO-, -C=NR₈, -O-, -S-, substituted or unsubstituted arylene, substituted or unsubstituted cycloalkylene, substituted or unsubstituted heterocyclylene, or -NR₈-, in any order;
X is selected from 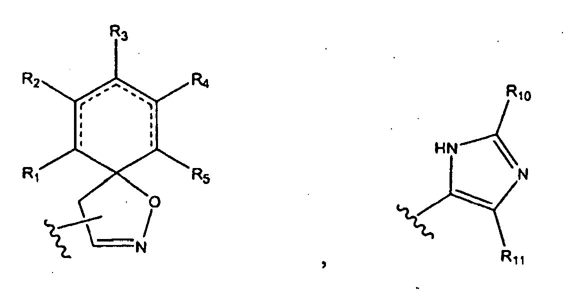 and -NH₂ ;
the dotted line represents one or two optional double bonds;
R₁ to R₅, R₁₀ and R₁₁ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRaRb or halogen;
R₆, R₇ and R₈ are independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} or halogen;
Rₐ and R_{b} are each independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, substituted or unsubstituted alkoxy, substituted or unsubstituted aryloxy or halogen;
t is 0, 1, 2 or 3;
or a tautomer, enantiomer, a pharmaceutically acceptable salt, a prodrug or a solvate thereof.

Preferably the linker contains one or more amide units of the type -CONRₐ- or -NRₐCO-, more preferably there are two units. Even more preferably the linker L means -CO-NH-(L')_{y}-NH-CO-, wherein y is selected from 2, 3, 4, 5, 6, 7, 8, 9 or 10 and L in between the amide units is formed by units as above defined.

In a preferred embodiment, R₂ and R₄ are -Br, R₃ is -OCH₃ and R₁ is OH.

In the above definition of compounds of formula (I) the following terms have the meaning indicated:
"Alkyl" refers to a straight or branched hydrocarbon chain radical consisting of carbon and hydrogen atoms, containing no saturation, having one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as a halo, hydroxy, alkoxy, carboxy, cyano, carbonyl, acyl, alkoxycarbonyl, amino, nitro, mercapto and alkylthio.
"Aryl" refers to a phenyl, naphthyl, indenyl, fenanthryl or anthracyl radical, preferably phenyl or naphthyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.
"Aralkyl" refers to an aryl group linked to an alkyl group. Preferred examples include benzyl and phenethyl.
"Cycloalkyl" refers to a stable 3- to 10-membered monocyclic or bicyclic radical which is saturated or partially saturated, and which consist solely of carbon and hydrogen atoms. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy and alkoxycarbonyl.
"Halogen" refers to bromo, chloro, iodo or fluoro.
"Heterocyclyl" refers to a stable 3- to 15 membered ring radical which consists of carbon atoms and from one to five heteroatoms selected from the group consisting of nitrogen, oxygen, and sulfur, preferably a 4- to 8-membered ring with one or more heteroatoms, more preferably a 5- or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

The terms "arylene", "cycloalkylene" and "heterocyclylene" refer to aryl, cycloalkyl and cycloalkyl groups having two free radicals.

The dotted line in the ring means that two double bonds can be present in the underlined part of said ring.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C1-6 alkanoyl group such as acyl and the like; carboxamido; alkyl groups including those groups having 1 to about 12 carbon atoms or from I to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "pharmaceutically acceptable salts, derivatives, solvates, prodrugs" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The term "prodrug" used in its broadest sense encompasses those derivatives that are converted in vivo to the compounds of the invention. Such derivatives would readily occur to those skilled in the art. Depending on the functional groups present in the molecule, the following derivatives of the present compounds are within the scope of the invention: esters, amino acid esters, phosphate esters, metal salts sulfonate esters, carbamates, and amides. Examples of well known methods of producing a prodrug of a given active compound are known to those skilled in the art and can be found e.g. in Krogsgaard-Larsen et al. "Textbook of Drug Design and Discovery" Taylor & Francis (April 2002).

The compounds of the invention may be in crystalline form either as free compounds or as solvates and it is intended that both forms are within the scope of the present invention. Methods of solvation are generally known within the art. Suitable solvates are pharmaceutically acceptable solvates. In a particular embodiment the solvate is a hydrate.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention. When a compound is drawn with explicit stereochemistry, it is intended to represent the racemic structure with relative stereochemistry as well as the enantiomers in different degrees of purity. In any case, the enantiomers and diastereoisomers of compounds represented with a particular stereochemistry form also part of the compounds of the invention.

The compounds Aerothionin, Homoaerothionin and 11,19-dideoxyfistularin, isolated from *Aplysina cavernicola*, are represented by formulae "Compound 3", "Compound 4" and "Compound 5": 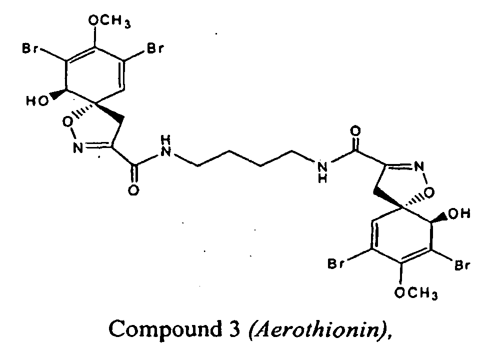 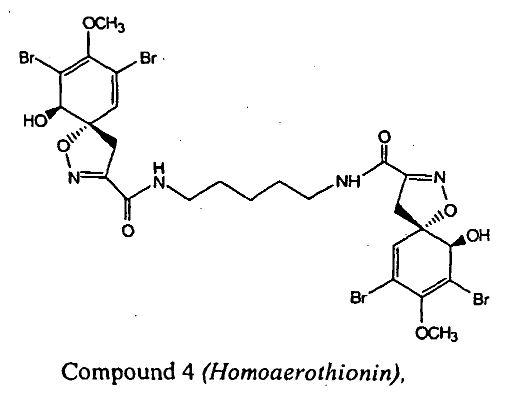 and

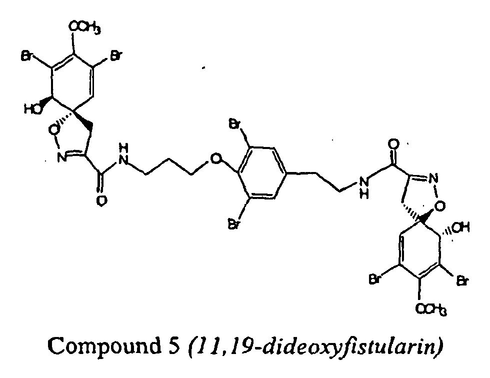

The compound 3 was described by L. Minale and G. Sodano. J. in J. Chem.Soc.Chem. Commun, 0, 1970, 751-753; whereas compound 4 was described in J.Chem.Soc.Perkin Trans, 0, 18-24, 1972 and the compound 5 was described by M.R. Kernan, R.C. Camhie in J. Nat. Prod. 1990,53,615-622. None of these documents discloses or suggests that these compounds presented VDCC blocker activity with eventually additional AChE and butyrylcholinesterase (BuChE) inhibitory activities.

From the mixture of *Aplysina fulva* and *Oceanapia* the following compounds have been isolated:

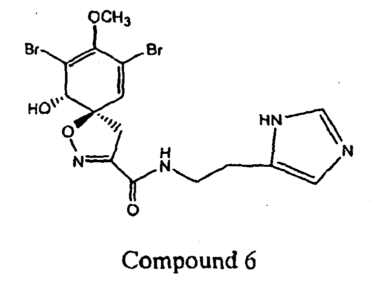

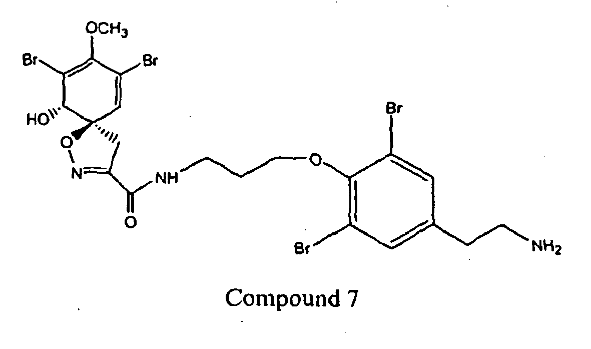

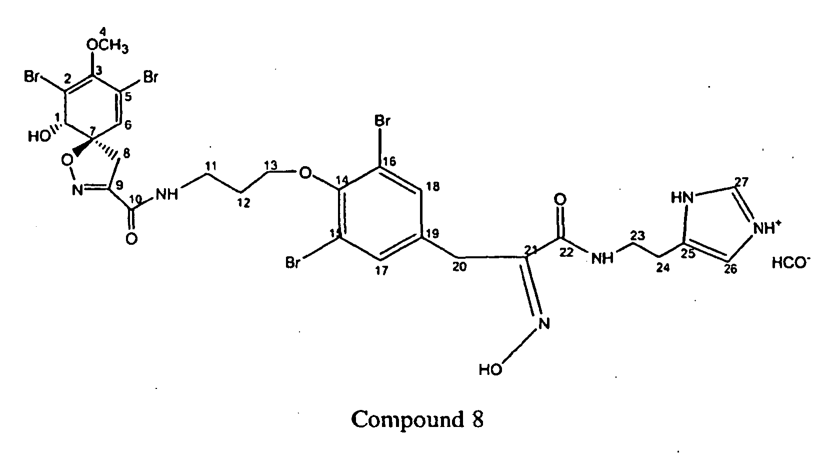

Compound 6 was firstly described by Cimino and G. Sodano, in Tetrahedron Letters, 24, 1983, 3029-3032 and compound 7 by Morris and Andersen, Canadian Journal Chemistry, 67, 1989, 677-681.

Compounds 7 and 8 were isolated by subjecting the isopropanolic fraction of the mixture of Aplysina fulva and Oceanopia to a VLC (vaccum-liquid chromatography), eluting with MeOH 100%, and subsequently performing a semi-preparative HPLC. It was isolated as its formic salt, in the form of a pale yellow syrup. Compound 8 is a new compound, and the experimental data which have allowed the elucidation of its structure are the following:
[α]²⁵ _{D}= -4.6 (c 0.26, MeOH), UV(MeOH) λₘₐₓ 220, 283nm.
¹H(400MHz) and ¹³C(100MHz), see **table 1;** ESI-MS[M+H⁺]⁺=864.84

**Table1. NMR Data for Compound 8 in CD₃OD**

| **Atom** **number** | **¹H** δ | **¹³C** δ |
|---|---|---|
| 1 | 4.09s | 75.52 |
| 2 | | 114.21 |
| 3 | | 149.31 |
| 4 | | 60.43 |
| 5 | | 122.75 |
| 6 | 6.39s | 132.28 |
| 7 | | 92.49 |
| 8a | 3.80d | 40.19 |
| 8b | 3.08d | |
| 9 | | 155.34 |
| 10 | | 161.57 |
| 11 | 3.56t | 38.02 |
| 12 | 2.08q | 30.63 |
| 13 | 4.02t | 72.23 |
| 14 | | 152.76 |
| 15 | | 118.76 |
| 16 | | 118.76 |
| 17 | 7.45s | 134.51 |
| 18 | 7.45s | 134.51 |
| 19 | | 137.42 |
| 20 | 3.81s | 28.82 |
| 21 | | 152.07 |
| 22 | | 165.52 |
| 23 | 3.52t | 39.67 |
| 24 | 2.86t | 26.70 |
| 25 | | 134.28 |
| 26 | 7.06s | 117.73 |
| 27 | 8.19s | 135.38 |

The present invention further provides a compound of formula: 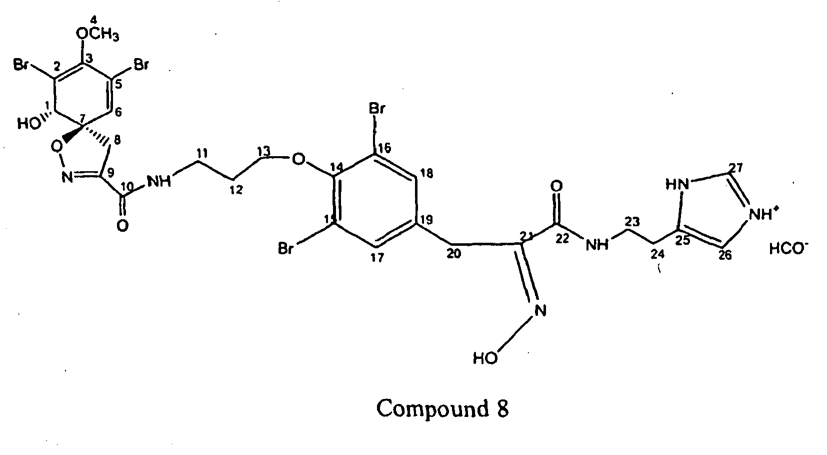 and pharmaceutical compositions comprising such a compound, or a pharmaceutically acceptable salt, derivative, prodrug as previously defined or stereoisomers thereof together with a pharmaceutically acceptable carrier, adjuvant, or vehicle, for administration to a patient. Although represented as the formiate salt, other pharmaceutically acceptable salts are also possible and are within the scope of the present invention.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositons may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day. It will be appreciated that it may be necessary to make routine variations to the dosage, depending on the age and condition of the patient, and the route of administration.

The compounds and compositions of this invention may be used with other drugs to provide a combination therapy. The other drugs may form part of the same composition, or be provided as a separate composition for administration at the same time or at different time.

The compounds of formula (I) defined above can be obtained by a convergent pathway strategy by coupling the two heterocyclic moieties which contain part of the linker. Synthetic procedures for preparing the compounds of formula (I) are described by Wasserman, H. et al. in J.Org. Chem. 1998, 63,5581-5586, and by Nishiyama S. et al. in Tetrahedron Letters, Vol.24, No. 32, pp. 3351-3352, 1983. The person skilled in the art of organic synthesis will readily design the process for each compound depending on the desired functionality of the heterocycles, the substituents and the nature of the linker to be obtained.

The reaction products may, if desired, be purified by conventional methods, such as crystallisation or chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in a pharmaceutical composition. In the case of salts and solvates the additional ionic and solvent moieties must also be non-toxic. The compounds of the invention may present different polymorphic forms, it is intended that the invention encompasses all such forms.

The compounds of formulas 1 to 5 defined above can also be obtained by isolation of the compound from the marine sponge Aplysina cavemicola, according to the following process which comprises the following steps:
a) effecting an extraction of a previously triturated *Aplysina cavernicola* with an organic solvent, in particular isopropanol
b) optionally concentrating the organic solvent extract and effecting a water/ether extraction of the product obtained in step a), and
c) effecting a column chromatography of the product obtained in the previous step in order to isolate the individual compounds.

The compounds of formulas 6 to 8 defined above may be obtained by isolation of the compound from a mixture of marine sponges *Aplysina fulva* and *Oceanapia*; in the case of Compound 6, it may be isolated according to the following process comprising the steps of:
a) effecting an extraction of a previously triturated mixture of *Aplysina fulva and oceanopia* with an organic solvent, in particular isopropanol
b) subjecting the isopropanol extract to a vacuum-liquid chromatography eluting with H₂O-MeOH 1:1; and
c) performing a semipreparative High Performance Liquid Chromatography.

Compounds 7 and 8 may be isolated by the following steps:
a) effecting an extraction of a previously triturated mixture of *Aplysina fulva and oceanopia* with an organic solvent, in particular isopropanol
b) subjecting the isopropanol extract to a vacuum-liquid chromatography eluting with MeOH 100%; and
c) performing a semipreparative High Performance Liquid Chromatography.

The invention is also directed to organic solvent extracts of *Aplysina cavernicola* and *Aplysina fulva* and *Oceanapia*; to said extracts for use as medicaments, in particular for the treatment of cognitive disorders as senile dementia, cerebrovascular dementia, mild recognition impairment, attention deficit disorder, and/or neurodegenerative dementing disease with aberrant protein aggregations as specially Alzheimers's disease or condition, stroke or prion disease as Creutzfeld-Jakob disease or Gerstmann-Straussler-Scheinker disease; to the use of said extracts in the manufacture of a medicament, in particular for the manufacture of a medicament for the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimer's disease or condition, or prion disease as reutzfeld-Jakob disease or Gerstmann-Straussler-Scheinker disease; and, finally, to a process for obtaining said extracts from *Aplysina cavernicola* and a mixture of *Aplysina fulva* and *Oceanapia,* as indicated above.

The following examples are given as further illustration of the invention, they should not be taken as a definition of the limits of the invention.

### EXAMPLES

The general procedures for the preparation of the compounds of the invention have been described above.

### Example 1: Isolation

### 1. Animal material:

a) *Aplysina cavernicola* was collected in 2001 at Ibiza sea (Spain). The sponge forms irregular straplike branches, the surface is conculose with fine protruding fibers, and it is flexible and very difficult to tear. The sponge is deep brownish-yellow in life and cream in preservative.
b) The mixture of *Aplysina fulva* and *Oceanapia* (minority) was collected in 2002 at 12 meters depth in Izabal, Guatemala. A voucher specimen (ORMA 006265) is deposited at PharmaMar, Colmenar Viejo, Madrid.

### 2. Extraction and Isolation :

a) A sample of frozen Aplysina cavemicola (600 gr wet weight) was cut into small pieces (2 cm) and triturated, the marine material was extracted with isopropanol (3L x 2). After decantation, the combined extracts were concentrated to give a brown solid (12 gr) and partitioned between diethyleter and water. The organic layer was concentrated to give a brown oil (1.67 gr). The oil was chromatographed on silica gel with a stepwise gradient solvent system: DCM/ MeOH (100:1), DCM/ MeOH (75:1), DCM/ MeOH (50:1), DCM/ MeOH (25:1). After this chromatography purification we isolated five compounds and all of them were described as Aeroplysinin-2 (compound **1),** Aeroplysinin-1 (compound **2),** Aerothionin (compound **3),** Homoaerothionin (compound **4),** and 11,19-dideoxyfistularin (compound **5).** 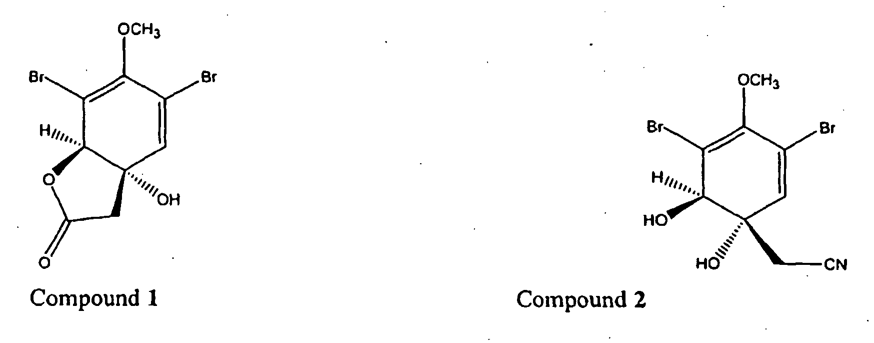 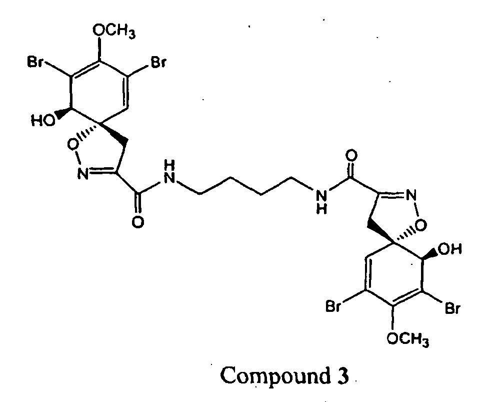 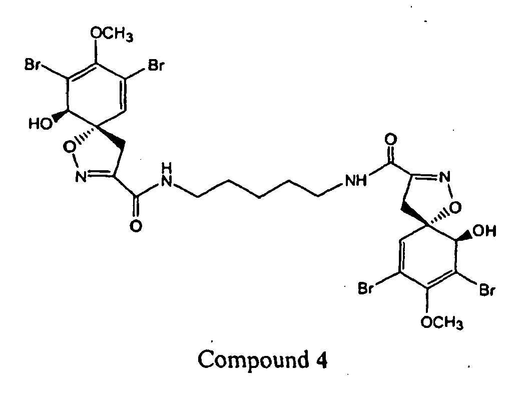 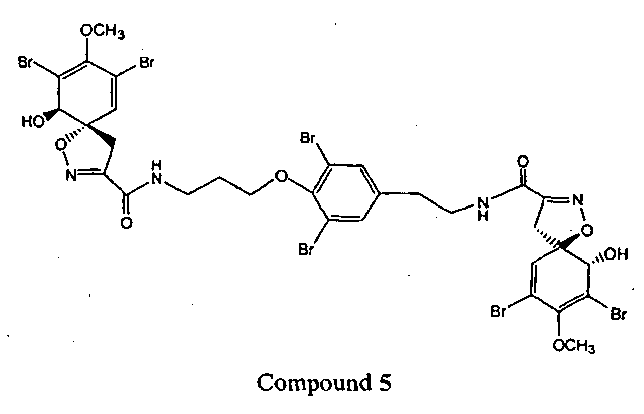
   The compound **1** was described by L. Minale and G. Sodano. J. Chem.Soc.Chem. Commun, 0, 1972, 674-675, the Compounds **2** and **3** were described by the same authors in J. Chem.Soc.Chem. Commun, 0, 1970, 751-753. The Compound **4** was described in J.Chem.Soc.Perkin Trans, 0, 18-24, 1972 and the compound **5** was described in M.R. Kernan, R.C. Camhie. J. Nat. Prod. 1990,53,615-622.
b) The mixture of *Aplysina fulva* and minority *Oceanapia* (158g) was diced and extracted with 2-propanol ( 3× 400ml). The combined extracts were concentrated to yield a crude of 6.42g. This material was subjected to VLC (vacuum-liquid chromatography) on Lichooprep RP-18, with a stepped gradient from H₂O to MeOH.
   14 mg of Compound 6 were isolated from the fraction eluting with H₂O-MeOH 1:1, followed by a subsequent semipreparative HPLC (High Performance Liquid Chromatography) (AtlantisSemiprep C-18, 10×150mm, gradient H₂O-AcN+0.1% formic acid from 20 to50% AcN in 30minUV detection at 254nm); this compound was disclosed by Cimino and G. Sodano., Tetrahedron Letters, 24, 1983, 3029-3032. 17.33mg of Compound **7** and 48.77mg of compound **8** were isolated from the same fraction, eluting with MeOH 100%, followed by a subsequent semipreparative HPLC (SymmetryPrep C-18, 19×300mm, gradient H₂O-AcN+0.1% formic acid from 20 to100% AcN in 30minUV detection at 254nm). Compound 7 was disclosed by Morris and Andersen, Canadian Journal Chemistry, 67, 1989, 677-681. Compound **8** is novel, and has been isolated as its formic salt, in the form of a yellow pail syrup. Experimental data:
   [α]²¹ _{D}= -4.6 (c 0.26, MeOH), UV(MeOH) λₘₐₓ 220, 283nm.
   ¹H(400MHz) and ¹³C(100MHz), see **table 1** above; ESI-MS[M+H⁺]⁺=864.84

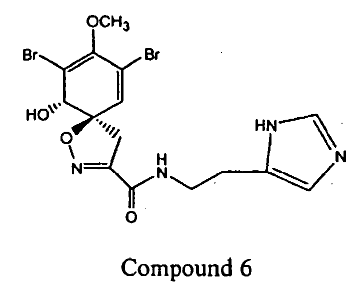

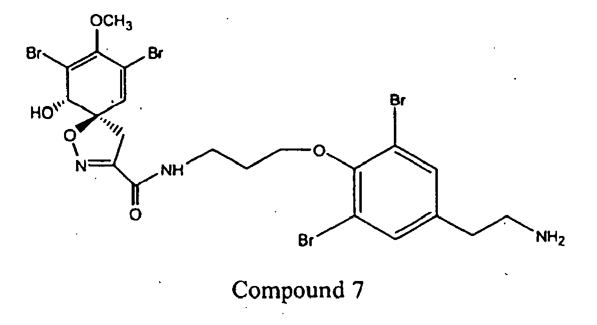

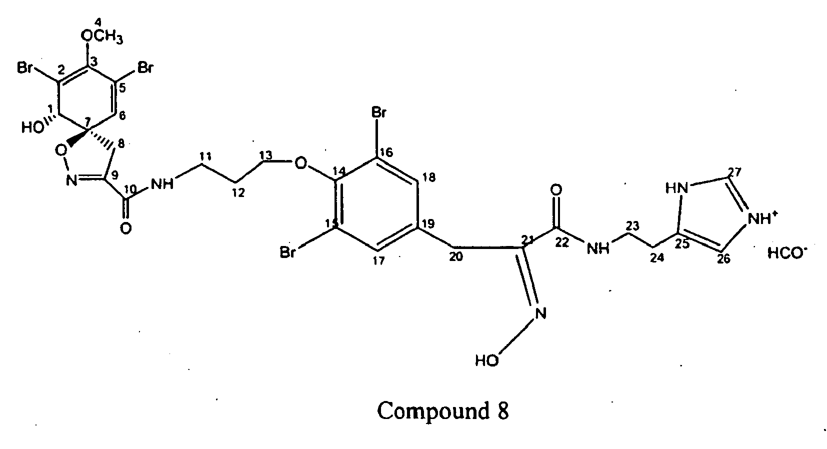

### 3. Materials and methods:

a) HPLC was perform with a symmetry C18 (4.6x150mm, 3.5µm) column using a Waters Alliance 2695 with a 2996 photodiode array detector and Waters ZQ2000 mass spectrometer used for the analytical separation and for UV and mass determination. The gradient used for the elution was 0-5 min 80 % A: 20%B; 5-40 min 100%B; 40-45 min 100%B; 45-46 min 80%A: 20%B (A= H2O 0.1% HF; B=AcN 0.1%HF).
   IR Spectra were recorded on a Bruker Tensor 27 FT-IR spectrometer.
   ¹H, ¹³C, NMR spectra were recorded on a Varian Mercury 400 MHz spectrometer in CD₃OD using the solvent as a reference standard (¹H, 4.87, 3.31, and ¹³C, 49.1). ¹H, ¹³C, COSY, HSQC, HMBC, NOE and DEPT spectra were obtained using standard Varian pulse sequences.
b) Mass spectral analyses were accomplished on a ZQ2000 single quadrupole mass spectrometer (Micromass) fitted with an ESI source and operated in the positive ionization mode.IR Spectra were recorded on a Bruker Tensor 27 FT-IR spectrometer.
   ¹H, ¹³C, NMR spectra were recorded on a Varian Mercury 400 MHz spectrometer in CD₃OD using the solvent as a reference standard (¹H, 4.87, 3.31, and ¹³C, 49.1). ¹H, ¹³C, COSY, HSQC, HMBC, NOE and DEPT spectra were obtained using standard Varian pulse sequences.

### Example 2: Biological Activity

### 1. Effects on VDCC

In order to find new marine compounds with VDCC blocker activity, we have performed a screening on SH-SY5Y neuroblastoma cells exposed to KCl 120 mM, The ability to block calcium channels is evaluated measuring cellular viability, using the LDH assay. Previously to the depolarization these cells were pretreated for 1 hour with the marine extracts at different concentrations (50, 15 and 5 µg/ml), 24 hours later survival is tested. Those compounds that exhibited a neuroprotective effect were selected and further assayed in SH-SY5Y cells loaded with Fluo-4 (Molecular probes) to measure their effects on changes in intracellular Ca²⁺ in response to depolarization with 60 mM KCI.
SH-SY5Y cells were plated at 5x10⁵ cells per well into Black / Clear Bottom 96-well culture plate, 48 hours before treatment. Cells were loaded with Fluo-4, 5 µM and pluronic acid, 0.1%, for 30 min at 37°C, 5%CO₂, following a incubation of 15 min at RT in Krebs-HEPES solution. Immediately cells are exposed to the samples at different concentrations for10 min. The marine extracts were tested, in Krebs-HEPES, at final concentrations of 50 µg/ml, 15 µg/ml and 5 µg/ml, and the isolated compounds at 10⁻⁵, 10⁻⁶ and 5x10⁻⁸ M. After the treatment, the fluorescence is measured in a Fluostar Optima plate reader (BMG) in response to depolarization with 60 mM KCl. The excitation wavelength was 485 nm, and that of emission 520 nm.
The marine extract from the sponge *Aplysina cavernicola* showed neuroprotective activity and VDCC blocker activity. Three compounds isolated from this extract have shown calcium channel blocking properties in SH-SY5Y neuroblastoma cells. Results are showed in table 2.

### 2. AChE Inhibition

AChE inhibitory activity was evaluated at 30°C by the colorimetric method reported by Ellman (Ellman, G.L. et al., 1961, Biochem Pharmacol, 7, 88-95). The assay solution consisted of 0.1 M phosphate buffer pH 8, 0.3 mM 5,5'-dithiobis (2-nitrobenzoic acid) (DTNB, Ellman's reagent), 0.02 units AChE (Sigma Chemical Co. from bovine erythrocytes), and 0.5 mM acetylthiocholine iodide as the substrate of the enzymatic reaction. The compounds tested were added to the assay solution and pre incubated with the enzyme for 5 min at 30°C. After that period, the substrate was added. The absorbance changes at 405 nm were recorded for 5 min with a microplate reader Digiscan 340T, the reaction rates were compared, and the percent inhibition due to the presence of test compounds was calculated. The reaction rate was calculated with, at least, triplicate measurements, and the percent inhibition due to the presence of test compound was calculated relative to the compound-free control. The results are shown in table 1.

### 3. BuChE inhibition

BuChE inhibitory activity was evaluated at 30°C by the colorimetric method reported by Ellman (Ellman, G.L. et al., 1961, Biochem Pharmacol, 7, 88-95). The assay solution consisted of 0.01 units BuChE from human serum, 0.1 M sodium phosphate buffer pH 8, 0.3 mM 5,5'-dithiobis (2-nitrobenzoic acid) (DTNB, Ellman's reagent), and 0.5 mM butyrylthiocholine iodide as the substrate of the enzymatic reaction. Enzyme activity was determined by measuring the absorbance at 405 nm during 5 minutes with a microplate reader Digiscan 340T. The tested compounds were preincubated with the enzyme for 10 minutes at 30°C. The reaction rate was calculated with, at least, triplicate measurements. The results are shown in table 2.

### 4. Toxicity measurement

The cytotoxicity effect of the molecules was tested in the human neuroblastoma cell line SH-SY5Y. These cells were cultured in 96-well plates in minimum essential medium, Ham's F12 medium, supplemented with 10% fetal bovine serum, 1% glutamine and 1% penicillin/streptomycin, and grown in a 5% CO₂ humidified incubator at 37°C. Cells were plated at 10⁴ cells for each well, at least, 48 hours before treatment. Cells were exposed for 24 hours to the compounds at different concentrations (from 10⁻⁵ to 10⁻⁹), quantitative assessment of cell death was made by measurement of the intracellular enzyme lactate dehydrogenase (LDH) (citotoxicity detection kit, Roche). The quantity of LDH was measured was evaluated in a microplate reader Anthos 2010, at 492 and 620 nm. Controls were taken as 100% viability. The results are shown in table 2.

### 5. Propidium competition

Propidium exhibits an increase in fluorescence on binding to AChE peripheral site, making it a useful probe for competitive ligand binding to the enzyme. Fluorescence was measured in a Fluostar Optima plate reader (BMG). Measurements were carried out in 100 µl solution volume, in 96-well plates. The buffer used was 1 mM Tris/HCl, pH 8.0. 5 µM AChE was incubated, at least 6 hours, with the molecule at different concentrations. 20 µM propidium iodide was added 10 min before fluorescence measurement. The excitation wavelength was 485 nm and that of emission 620 nm. *In vitro* results are showed in table 2.

### 6. Antioxidant activity

In order to evaluate if these compounds have antioxidant properties, SH-SY5Y cells were exposed for 24 hours to 100 µM H₂O₂, previously these cells were pretreated for 1 hour with the molecules at different concentrations (from 10⁻⁵ to 10⁻⁹). The antioxidant activity is evaluated measuring cellular viability, using the LDH assay. Cells SH-SY5Y were cultured in 96-well plates as toxicity experiments. The results are shown in table 2.

**TABLE 2**

| **Compound** | **% inhibition** | **% inhibition** | **%** | **Toxicity** | **Propidium** | **Antioxidant** |
|---|---|---|---|---|---|---|
| | **VDCC** | **AChE** | **inhibition** | **(µM)** | **competition** | **activity** |
| | **(concentration)** | **(concentration)** | **BuChE** | | **(30** µ**M)** | |
| | | | **(30** µ**M)** | | | |
| **1** | 0 | 7±1 | 0 | 0.6 | NO | NO |
| | | (3 x 10⁻⁵ M) | | | | |
| **2** | 0 | 19±3 | 0 | 6 | NO | NO |
| | | (3 x 10⁻⁵ M) | | | | |
| **3** | 75±17 | 12±1 | 13±1 | 18 | NO | NO |
| | (1,8 x 10⁻⁵ M) | (3 x 10⁻⁵ M) | | | | |
| **4** | 95±4 | 9±4 | 10±1 | 6 | YES | NO |
| | (1,8 x 10⁻⁵ M) | (3 x 10⁻⁵ M) | | | | |
| | | | | | | |
| **5** | 34±14 | 63±3 | 57±7 | >100 | YES | NO |
| | (1,8 x 10⁻⁵ M) | (3 x 10⁻⁵ M) | | | | |
| **6** | 18 ± 10 | 0 | / | Not toxic | / | NO |
| | (10⁻⁵ M) | (10⁻⁴ M) | | at 10 µM | | |
| **7** | 67 ± 14.5 | 9.5 ± 3 | / | Not toxic | / | NO |
| | (10⁻⁵ M) | (10⁻⁴ M) | | at 10 µM | | |
| | | 5 ± 3 | | | | |
| | | (10⁻⁵ M) | | | | |
| **8** | 96 ± 3 | 0 | / | Not toxic | / | NO |
| | (10⁻⁵ M) | (10⁻⁴ M) | | at 10 µM | | |
| | 48 | | | | | |
| | (10⁻⁶ M) | | | | | |
| | | | | | | |
| | 9 | | | | | |
| | (10⁻⁷ M) | | | | | |

## Claims

1. Use of a compound of formula **I:** 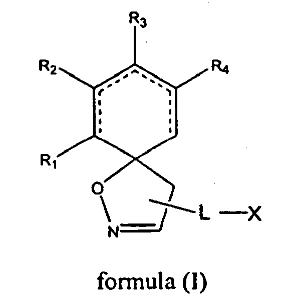 wherein
L is a linker, consisting of a lineal sequence of 3-20 units selected from the group formed by -CR₆R₇-,-CR₆=, =CR₆-, -CO-, -C=NR₈, -O-, -S-, substituted or unsubstituted arylene, substituted or unsubstituted cycloalkylene, substituted or unsubstituted heterocyclylene, or -NR₈-, in any order;
X is selected from 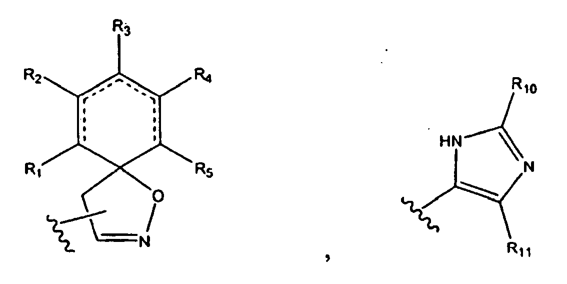 and -NH₂ ;
the dotted line represents one or two optional double bonds;
R₁ to R₅, R₁₀ and R₁₁ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRaRb or halogen;
R₆, R₇ and R₈ are independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} or halogen;
Rₐ and R_{b} are each independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, substituted or unsubstituted alkoxy, substituted or unsubstituted aryloxy or halogen;
t is 0, 1, 2 or 3;
or a tautomer, enantiomer, a pharmaceutically acceptable salt, a prodrug - wherein prodrug referes to esters, amino acid esters, phosphate esters, metal salts sulfonate esters, carbamates, and amide derivative - or a solvate thereof;
in the manufacture of a medicament for the treatment of cognitive and neurodegenerative diseases or disorders.

2. Use according to claim 1, wherein the cognitive and neurodegenerative diseases or disorders are selected from the gorup formed by brain ischemia, stroke, senile dementia, cerebrovascular dementia, mild recognition impairment, attention deficit disorder, neurodegenerative dementing disease with aberrant protein aggregations, preferably Alzheimer's Disease or condition, prion disease as Creutzfeld-Jakob disease or Gerstmann-Straussler-Scheinker disease.

3. Use according to any of claims 1 and 2, wherein the linker that connects the spirocycle unit and group X comprises one or more units selected from -CONRₐ- or -NRₐCO-.

4. Use according to any of claims 1-3 wherein the linker L means:
-CO-NH-(L)_{y}-NH-CO-, wherein y is selected from 2, 3, 4, 5, 6, 7, 8, 9 or 10 and L' is formed of the same units as defined above for L.

5. Use according to any of claims 1-4, wherein R₂ and R₄ are -Br, R₃ is -OCH₃ and R₁ is OH.

6. Use according to any of claims 1-5 wherein the compound of formula I is selected from the group formed by the compounds: 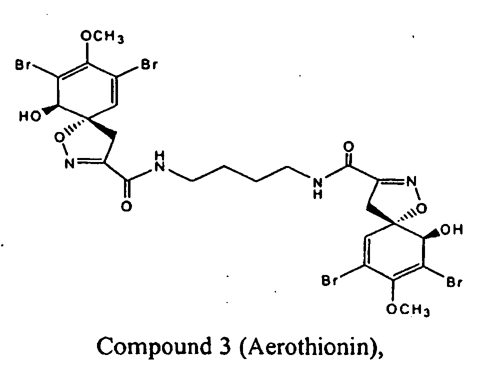 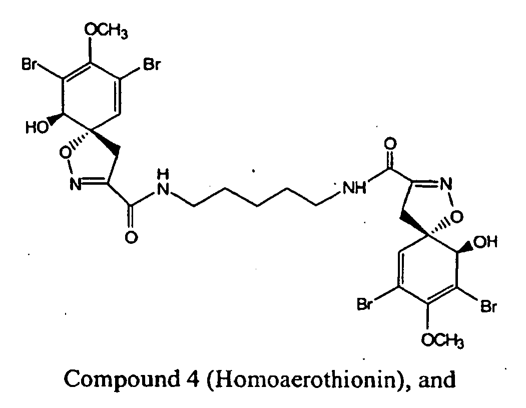 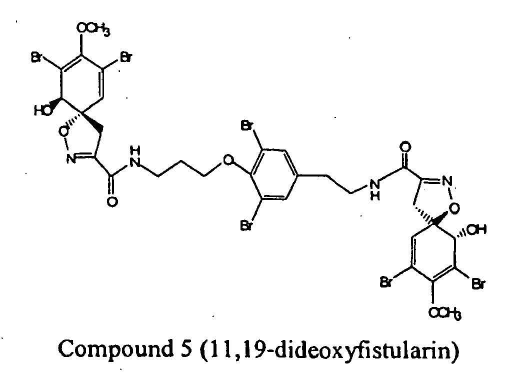 or their enantiomers, diastereomers, tautomers, and pharmaceutically acceptable salts thereof.

7. Use according to any of claims 1-3 wherein the compound of formula I is selected from the group formed by the compounds: 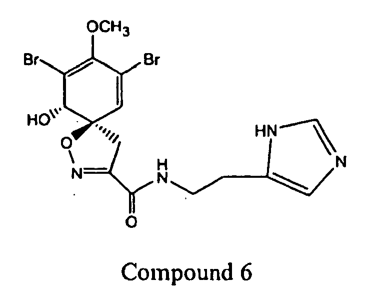 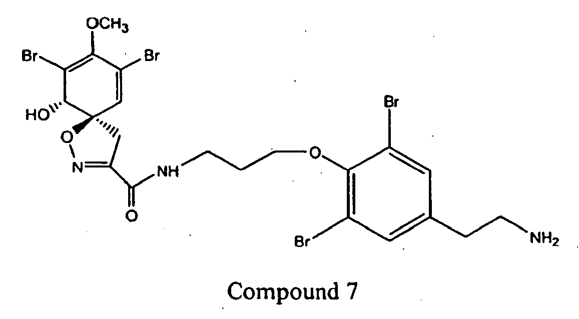 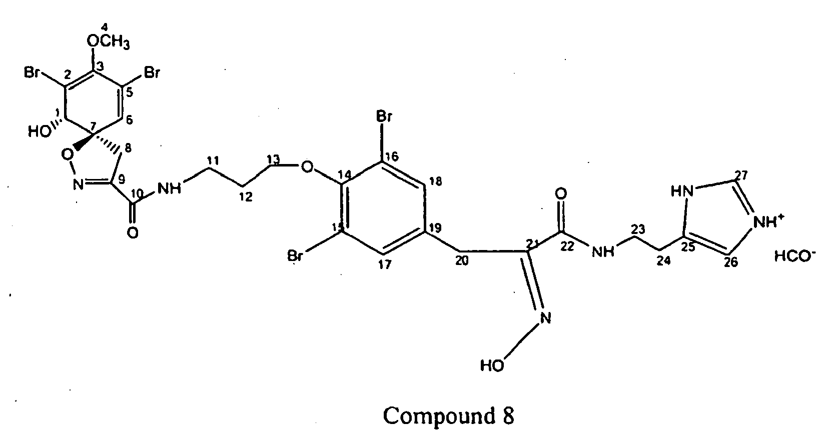 or their enantiomers, diastereomers, tautomers, and pharmaceutically acceptable salts thereof.

8. A compound of formula 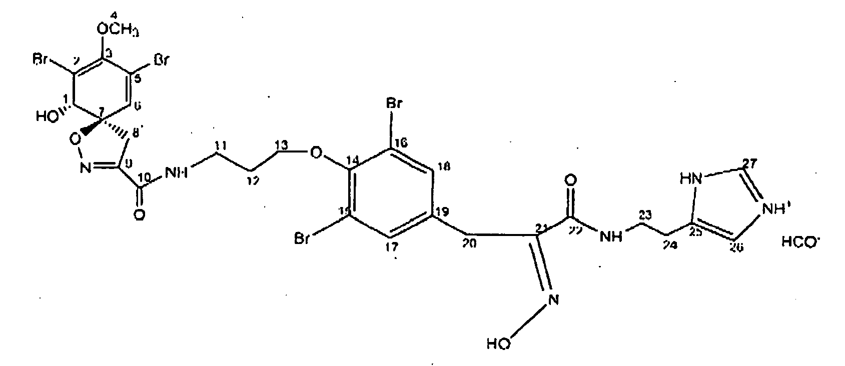 or its enantiomers, diastereomers, tautomers, and pharmaceutically acceptable salts thereof.

9. A pharmaceutical composition which comprises the compound defined in claim 8, or a tautomer, a pharmaceutically acceptable salt, a prodrug or a solvate thereof, together with a pharmaceutically acceptable carrier, adjuvant or vehicle, wherein the prodrug is as defined in claim 1.

10. A process for the isolation of the compound defined in claim 8, which comprises the following steps:
a) effecting an extraction of a previously triturated mixture of *Aplysina fulva and oceanopia* with an organic solvent, preferably isopropanol
b) subjecting the isopropanol extract to a vacuum-liquid chromatography eluting with McOH 100%; and
c) performing a semipreparative High Performance Liquid Chromatography.

## Patentansprüche

1. Verwendung einer Verbindung der Formel I: 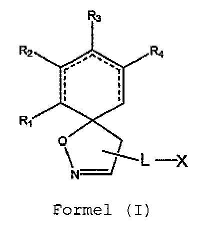 wobei
L einen Linker darstellt, der aus einer linearen Sequenz von 3 - 20 Einheiten ausgewählt aus der aus -CR₆R₇-, -CR₆=, =CR₆-, -CO-, -C=NR₈, -O-, -S-, substituiertem oder unsubstituiertem Arylen, substituiertem oder unsubstituiertem Cycloalkylen, substituiertem oder unsubstituiertem Heterocyclylen oder -NR₈- gebildeten Gruppe in beliebiger Anordnung besteht;
X ausgewählt ist aus 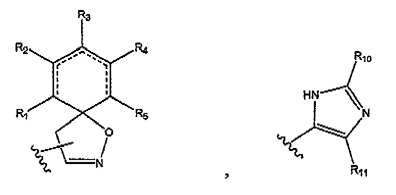 und -NH₂;
die gestrichelte Linie eine oder zwei optionale Doppelbindung(en) darstellt;
R₁ bis R₅, R₁₀ und R₁₁ unabhängig ausgewählt sind aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Heterocyclyl, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} oder Halogen;
R₆, R₇ und R₈ unabhängig ausgewählt sind aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Aralkyl, substituiertem oder unsubstituiertem Heterocyclyl, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} oder Halogen;
Rₐ und R_{b} unabhängig ausgewählt sind aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Aralkyl, substituiertem oder unsubstituiertem Heterocyclyl, substituiertem oder unsubstituiertem Alkoxy, substituiertem oder unsubstituiertem Aryloxy oder Halogen;
t 0, 1, 2 oder 3 darstellt;
oder eines Tautomers, Enantiomers, eines pharmazeutisch akzeptablen Salzes, eines Pro-Pharmakons, wobei sich Pro-Pharmakon auf Ester, Aminosäureester, Phosphatester, Metallsalzsulfonatester, Carbamate und Aminderivate bezieht, oder eines Solvats davon;
bei der Herstellung eines Medikaments zur Behandlung kognitiver und neurodegenerativer Krankheiten oder Störungen.

2. Verwendung nach Anspruch 1, wobei die kognitiven und neurodegenerativen Krankheiten oder Störungen ausgewählt sind aus der aus Hirnischämie, Hirnschlag, seniler Demenz, zerebrovaskulärer Demenz, leichter Wahrnehmungsbeeinträchtigung, Aufmerksamkeits-Defizit-Syndrom, neurodegenerativer Demenzkrankheit mit aberrierenden Proteinablagerungen, hauptsächlich Alzheimersche Krankheit oder Verfassung, Prionenkrankheit wie Creutzfeld-Jakob-Krankheit oder Gerstmann-Straussler-Scheinker-Krankheit gebildeten Gruppe.

3. Verwendung nach einem der Ansprüche 1 und 2, wobei der die spirocyclische Einheit und Gruppe X verbindende Linker eine oder mehr Einheiten ausgewählt aus -CONRₐ-, oder -NRₐCO- umfasst.

4. Verwendung nach einem der Ansprüche 1-3, wobei der Linker L bedeutet:
- CO-NH-(L')y-NH-CO-, wobei y ausgewählt ist aus 2, 3, 4, 5, 6, 7, 8, 9 oder 10 und L' aus den gleichen Einheiten wie oben für L definiert gebildet ist.

5. Verwendung nach einem der Ansprüche 1-4, wobei R₂ und R₄ -Br darstellen, R₃ -OCH₃ darstellt und R₁ OH darstellt.

6. Verwendung nach einem der Ansprüche 1-5, wobei die Verbindung der Formel I ausgewählt ist aus der Gruppe gebildet aus den Verbindungen: 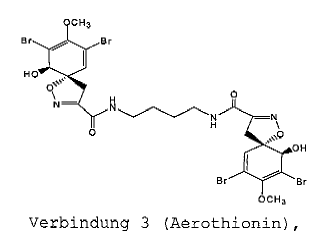 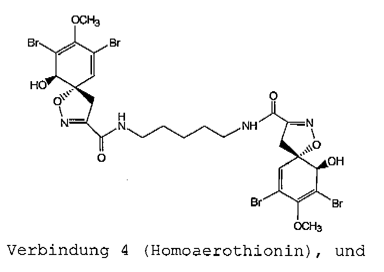 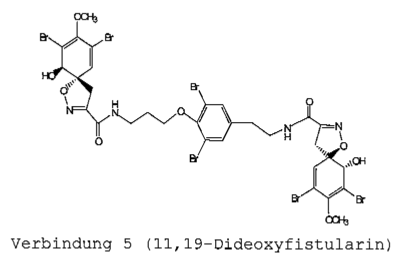 oder Enantiomeren, Diastereomeren, Tautomeren und pharmazeutisch akzeptablen Salzen davon.

7. Verwendung nach einem der Ansprüche 1-3, wobei die Verbindung der Formel I ausgewählt ist aus der Gruppe gebildet aus den Verbindungen: 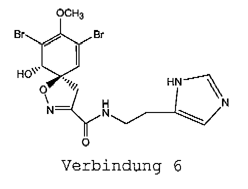 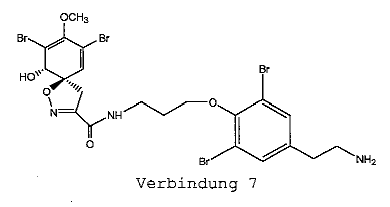 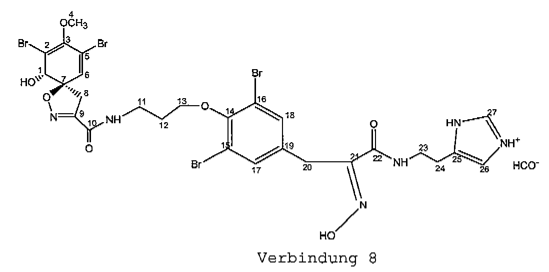 oder Enantiomeren, Diastereomeren, Tautomeren und pharmazeutisch akzeptablen Salzen davon.

8. Verbindung der Formel 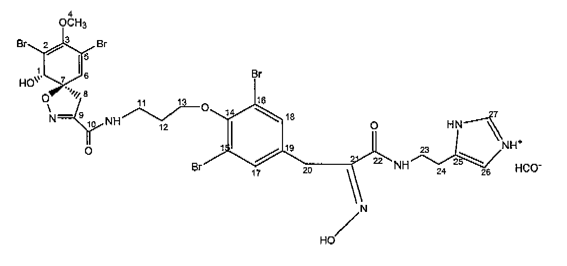 oder Enantiomere, Diastereomere, Tautomere und pharmazeutisch akzeptable Salze davon.

9. Pharmazeutische Zusammensetzung, welche die in Anspruch 8 definierte Verbindung umfasst, oder ein Tautomer, ein pharmazeutisch akzeptables Salz, ein Pro-Pharmakon oder ein Solvat davon, zusammen mit einer/einem pharmazeutisch akzeptablen Trägersubstanz, Hilfsstoff oder Vehikel, wobei das Pro-Pharmakon wie in Anspruch 1 definiert ist.

10. Verfahren zur Isolierung einer in Anspruch 8 definierten Verbindung, welches die folgenden Schritte umfasst:
a) Ausführen einer Extraktion einer zuvor verriebenen Mischung von *Aplysina fulva* und *oceanopia* mit einem organischen Lösungsmittel, bevorzugt Isopropanol,
b) Unterwerfen des Isopropanol-Extrakts einer mit 100% MeOH eluierenden Vakuum-Flüssigchromatographie; und
c) Durchführen von semipräparativer Hochleistungsflüssigkeitschromatographie (HPLC).

## Revendications

1. Utilisation d'un composé de formule I : 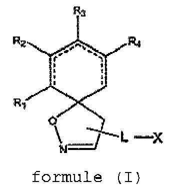 dans laquelle
L représente un groupe de liaison, consistant en une séquence linéaire de 3 à 20 motifs choisis dans le groupe formé par les motifs -CR₆R₇-, -CR₆=, =CR₆, -CO-, -C=NR₈, -O-, -S-, arylène substitué ou non substitué, cycloalkylène substitué ou non substitué, hétérocyclylène substitué ou non substitué et -NR₈-, dans n'importe quel ordre ;
X est choisi entre des groupes 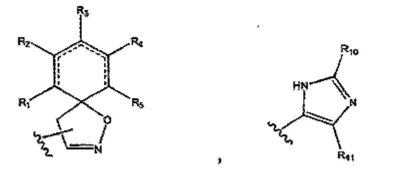 et **-NH₂;**
la ligne discontinue représente une ou deux doubles liaisons facultatives ;
R₁ à R₅, R₁₀ et R₁₁ sont choisis indépendamment entre un atome d'hydrogène, des groupes alkyle substitué ou non substitué, cycloalkyle substitué ou non substitué, alcényle substitué ou non substitué, aryle substitué ou non substitué, hétérocyclyle substitué ou non substitué, -CORₐ, -C(O)ORₐ, - OC(O)Rₐ, -C(O)NRₐR_{b}, -C=NRₐ, -CN, -ORₐ, -S(O)ₜ-Rₐ, -NRₐR_{b}, - NRₐC(O)R_{b}, -NO₂, -N=CRₐR_{b} et halogéno ;
R₆, R₇ et R₈ sont choisis indépendamment entre un atome d'hydrogène, des groupes alkyle substitué ou non substitué, cycloalkyle substitué ou non substitué, alcényle substitué ou non substitué, aryle substitué ou non substitué, aralkyle substitué ou non substitué, hétérocyclyle substitué ou non substitué, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐ(O)R_{b}, -NO₂, -N=CRₐR_{b} et halogéno ;
Rₐ et R_{b} sont choisis chacun indépendamment entre un atome d'hydrogène, des groupes alkyle substitué ou non substitué, cycloalkyle substitué ou non substitué, alcényle substitué ou non substitué, aryle substitué ou non substitué, aralkyle substitué ou non substitué, hétérocyclyle substitué ou non substitué, alkoxy substitué ou non substitué, aryloxy substitué ou non substitué et halogéno ;
t est égal à 0, 1, 2 ou 3 ;
ou d'une de ses formes tautomères, d'un de ses énantiomères, d'un de ses sels pharmaceutiquement acceptables, d'un précurseur médicamenteux, le terme "précurseur médicamenteux" désignant des esters, des esters d'aminoacides, des esters du type phosphate, des esters du type sulfonate des sels métalliques, des carbamates et des dérivés du type amide, ou bien d'un de leurs produits de solvatation ;
dans la production d'un médicament destiné au traitement de maladies ou troubles cognitifs et neurodégénératifs.

2. Utilisation suivant la revendication 1, dans laquelle les maladies ou troubles cognitifs et neurodégénératifs sont choisis dans le groupe formé par l'ischémie cérébrale, un ictus, la démence sénile, la démence vasculaire cérébrale, l'altération cognitive bénigne, un trouble de déficit d'attention, une maladie du type démence neurodégénérative présentant des agrégations de protéines aberrantes, de préférence la maladie ou l'affection d'Alzheimer, une maladie à prions telle que la maladie de Creutzfeld-Jakob ou la maladie de Gertsmann-Straussler-Scheinker.

3. Utilisation suivant l'une quelconque des revendications 1 et 2, dans laquelle le groupe de liaison qui connecte le motif spirocyclique et le groupe X comprend un ou plusieurs motifs choisis entres des motifs CONRₐ et -NRₐCO-.

4. Utilisation suivant l'une quelconque des revendications 1 à 3, dans laquelle le groupe de liaison L représente un groupe :
-CO-NH-(L')_{y}-NH-CO-, dans lequel y est choisi entre 2, 3, 4, 5, 6, 7, 8, 9 ou 10 et L' est formé des mêmes motifs définis ci-dessus pour L.

5. Utilisation suivant l'une quelconque des revendications 1 à 4, dans laquelle R₂ et R₄ représentent des groupes -Br, R₃ représente un groupe -OCH₃ et R₁ représente un groupe OH.

6. Utilisation suivant l'une quelconque des revendications 1 à 5, dans laquelle le composé de formule I est choisi dans le groupe formé par les composés : 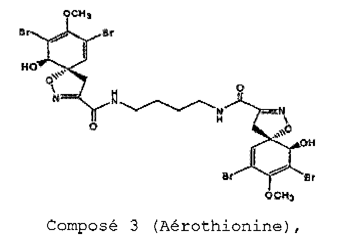 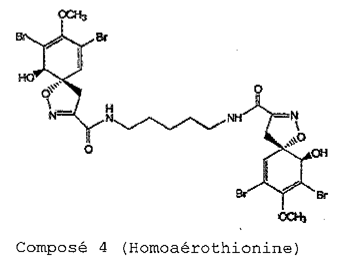 et 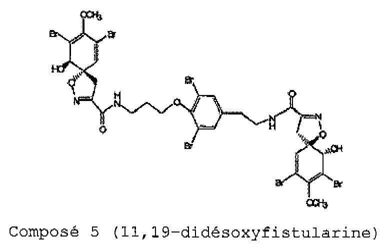 ou leurs énantiomères, leurs diastéréoisomères, leurs formes tautomères et leurs sels pharmaceutiquement acceptables.

7. Utilisation suivant l'une quelconque des revendications 1 à 3, dans laquelle le composé de formule I est choisi dans le groupe formé par les composés : 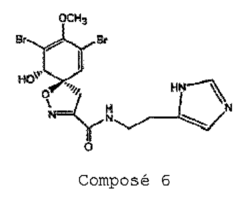 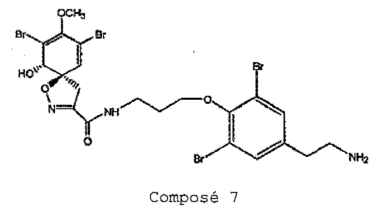 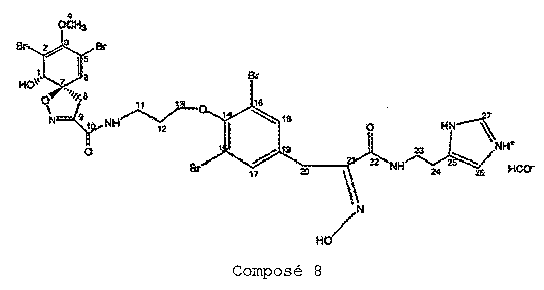 ou leurs énantiomères, leurs diastéréoisomères, leurs formes tautomères et leurs sels pharmaceutiquement acceptables.

8. Un composé de formule 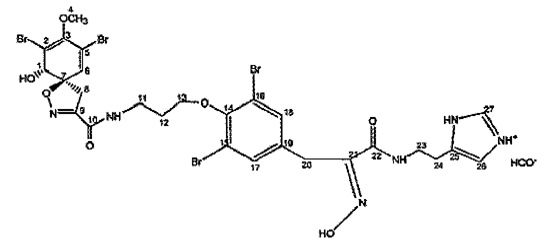 ou ses énantiomères, ses diastéréoisomères, ses formes tautomères et ses sels pharmaceutiquement acceptables.

9. Une composition pharmaceutique qui comprend le composé défini dans la revendication 8, ou une de ses formes tautomères, un de ses sels pharmaceutiquement acceptables, un de ses précurseurs médicamenteux ou un de ses produits de solvatation, conjointement avec un support, adjuvant ou véhicule pharmaceutiquement acceptables, le précurseur médicamenteux étant tel que défini dans la revendication 1.

10. Un procédé pour isoler un composé défini dans la revendication 8, qui comprend les étapes suivantes :
a) effectuer une extraction d'un mélange, préalablement trituré, d'*Aplysina fulva* et *oceanopia* avec un solvant organique, de préférence l'isopropanol,
b) soumettre la phase d'extraction à l'isopropanol à une chromatographie en phase liquide sous vide en éluant avec 100 % de MeOH ; et
c) mettre en oeuvre une chromatographie en phase liquide à hautes performances semi-préparative.
